# EUROPEAN PATENT APPLICATION

(11) **EP 3 671 755 A1**
(43) Date of publication of application: **24.06.2020**
(21) Application number: 18382945.6
(22) Date of filing: 19.12.2018
(51) Int. Cl.: G16H 20/90

(54) **METHOD AND SYSTEM FOR GENERATING TREATMENT PLANS FOR PSYCHOLOGICAL ILLNESS AND DISORDERS**

(71) Applicant: Telefonica Innovacion Alpha S.L, 28050 Madrid (ES)
(72) Inventor: LANTZ, Johan, 28050 MADRID (ES)
(74) Representative: Herrero & Asociados, S.L.

(57) **Abstract**

A system and method implemented based on four hierarchical layers, a static layer, a data modelling layer, an application logic layer and a visualisation layer, for generating treatment plans of psychological illness and disorders, comprising:
a content editor (10) at the static layer for generating a file containing data static content which declares, using a machine readable format, a treatment plan defined by a therapist for a patient;
the data modelling layer comprising means for reading the static content of the file and creating, at, based on the static content, a logical model with which a computer program works;
the application logic layer comprising means for generating events associated with patient's inputs triggered for the static content, the events being used to create dynamic content to be stored in the content storage (20) or used to modify dynamically the logical model;
the visualisation layer comprising a user interface for presenting the logical model and receiving inputs.

## Description

### Field of the invention

The present invention has its application within the telecommunication sector, more specifically, relates to the deployment of tools in telecommunication network entities (e.g., applications in mobile user terminals such as smartphones or tablets, webpages, etc.) which work systematically with a user/patient in improving his mental condition, with or without the direct interaction of a therapist, for the treatment of mental illness and disorders.

This invention describes a technical framework that can be used to design, evolve and deploy treatment programs informatically as well as collect and evaluate the patient's progress inside said program over time.

More particularly, the present invention refers to a system and method for generating a computer program or application to build treatment plans of psychological illness and disorders.

### Background of the invention

Developing one computer program based system or application (app) focused on a single mental disorder can easily take a team of engineers over a year to complete and that is just for one specific case. This means that even if focusing only on primary disorders such as depression and anxiety, the time to market for any single treatment plan/app is extremely time consuming and costly. It also has the consequence that less frequent disorders, which cause equal or larger strain on individuals and society, will end up last in the priority queue with only a small chance of ever reaching the hands of end users.

The main problem with an app/computer-program based approach is that each program and plan needs to be addressed based on its unique challenges and conditions. In turn, this leads to an approach where each disorder requires its own dedicated App, which is hard and costly to be scaled, as mentioned before.

Another problem is that the engineers building the system does not speak the same language as the doctors needed to design the content and so reaching a common ground understanding each others' challenges takes time and is, again, impossible to scale. Each team of engineers and each doctor face the same initial challenges, making the project too expensive to maybe even have a chance to launch and much less to evolve and be maintained.

US 2008/0103833 A1 discloses a system for populating patient records by use of evidence-based relational database, which compares the medical practitioners diagnoses to predetermined responses, to produce accurate patient chart notes and the integration of stored and generated data into clinical and administrative medical record keeping and billing. Episodic encounters are developed into cases for a specific patient under the care of a practitioner. The subjective symptoms from the patient and the objective observations of the care provider concurrent with the episode are used to form a diagnosis which presents a treatment regimen from an evidence-based relational database and populates medical and administrative templates. Patient history and updated information are retained in the database and "best practice" treatment plans are continually placed in the relational database from practice guides and experts in the field.

US 6289513 B1 is focused on sentence of natural language generation and discloses a method and system for generating an application, using a plurality of components, each component defining an application block. US 6289513 B1 describes a plurality of user-defined application-specific properties, each property being associated with one of the plurality of components, and storing based on a non-programmatic user input and receiving structured data input via a questionnaire, based at least in part on the plurality of components. Inputs are referred to data preferably entered via a hierarchical questionnaire data input mechanism, which are not predefined lists. The structured questionnaire is converted into text. Text is generated based, at least in part, on the structured data, including dynamic runtime generation of a plurality of simple sentences from a plurality of sub-sentence segments based, at least in part, on user input, based at least in part on the components and providing an application based on at least some of the plurality of user-defined application-specific properties and on the associated components. However, although texts are automatically generated when the users answers something, these texts do not reference the answers between exercises/questionnaires.

Therefore, there is a need in the state of the art for developing treatment plans of psychological illness or disorders which are effective to be replicated and scaled.

### Summary of the invention

The present invention solves the aforementioned problems and overcomes previously explained state-of-art work limitations by providing a method to.

This invention presents a way to build a framework for developing clinical and non clinical treatment plans in the form of computer programs, based on a generic approach that can be reused to a large extent between different types of mental illnesses and disorders.

Furthermore, the invention allows a complete isolation and externalization of the program content creation, translation, etc., by fully separating the technical side of developing the App or computer program from the clinical side of developing the treatment plan.

A first aspect of the present invention refers to a computer-implemented method for generating treatment plans of psychological illness and disorders, which comprises the following steps:
- at a static layer, generating, a file containing data static content, which declares, using a machine readable format, a treatment plan defined by a therapist for a patient;
- at a data modelling layer, reading the static content of the file and creating a logical model, based on the static content, model with which a computer program works;
- at an application logic layer, generating events associated with inputs from the patient which are triggered for the static content defined at the static layer, the events being used to create dynamic content which is either stored or used to modify dynamically the logical model created at the data modelling layer;
- at a visualisation layer, presenting the logical model to the patient and receiving the inputs from the patient.

A second aspect of the present invention refers to a system configured to implement the method described before by comprising a processor implementing four layers, a visualisation layer built on an application logic layer which is built on an a data modelling layer, in turn, built on a static layer, and a content storage connected to the processor, the processor further comprising:
- a content editor with means for generating, at the static layer, a file containing data static content which declares a treatment plan using a machine readable format, the treatment plan defined by a therapist for a patient;
- means for reading the static content of the file and creating, at the data modelling layer, based on the static content, a logical model with which a computer program works;
- means for generating, at the application logic layer, events associated with inputs from the patient which are triggered for the static content defined at the static layer, the events being used to create dynamic content which is either stored in the content storage or used to modify dynamically the logical model created at the data modelling layer;
- a user interface, at the visualisation layer, for presenting the logical model to the patient and receiving the inputs from the patient.

The method and system in accordance with the above described aspects of the invention has a number of advantages with respect to the aforementioned prior art, which can be summarized as follows:
- The invention provides doctors and experts with a (four-layer architecture based) platform of tools to develop new dynamic treatment plans without the technical barriers and costs of writing software code and instead of building dedicated applications for each disease or disorder.
- With this platform in the hands of experts and doctors all over the world, cutting edge treatments can reach the end-user in a scale and at a speed that is unprecedented.
- By introducing the dynamic processing of the static content the treatment evolves from the linear "textbook" format to a dynamic format that is adapted to the users input and progress, by dynamically generating new cards, input options and content based on patient's answers. The system is referred to old answers to create dynamic content with new questions, digging deeper into the patients' problems.
- The four-layered architecture allows for a very high level of reusability making the time and cost going to market with new treatments much faster and cheaper.
- Regarding the aforementioned US2008/0103833, US2008/0103833 focuses on electronic handling of patient records and the generation of such records in an automatic fashion, being more particularly focused on the storage and collection of existing information, but not at all deal with the case of treating a mental problem.
- Regarding the aforementioned US6289513, US6289513 is focused on building a readable content based on the user input, while the present invention is focused on tailoring and modifying the treatment plan for the patient based on the user selections. US6289513 does not disclose generating new cards, injecting new options in lists, etc.

These and other advantages will be apparent in the light of the detailed description of the invention.

### Description of the drawings

For the purpose of aiding the understanding of the characteristics of the invention, according to a preferred practical embodiment thereof and in order to complement this description, the following Figures are attached as an integral part thereof, having an illustrative and non-limiting character:
Figure 1 shows a schematic high level structure of a system for generating a treatment plan for patients of psychological illness and disorders, according to a possible embodiment of the invention.
Figure 2 shows an example of exercise reuse across treatment programs.
Figure 3 shows a visualization of a card generated for collecting free-text in a textarea input by a patient.
Figure 4 shows a visualization of content for a treatment program statically declared as a checklist.
Figure 5 shows a visualization of a dynamically generated card where the patient selects two options.
Figure 6 shows a visualization of a first dynamically generated card based on a selected first option.
Figure 7 shows a visualization of a second dynamically generated card based on a selected second option.
Figure 8 shows a visualization of a static card with dynamically injected checklist options for a second checklist.

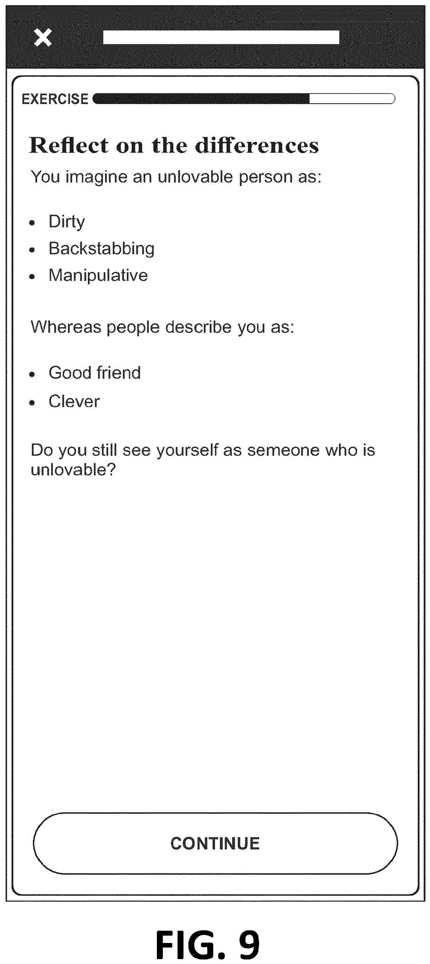

Figure 9 shows a visualization of replies injected into a generated card.
Figure 10 shows a flow diagram of a method for generating a treatment plan for patients of psychological illness and disorders, according to a possible embodiment of the invention.

### Preferred embodiment of the invention

The matters defined in this detailed description are provided to assist in a comprehensive understanding of the invention. Accordingly, those of ordinary skill in the art will recognize that variation changes and modifications of the embodiments described herein can be made without departing from the scope and spirit of the invention. Also, description of well-known functions and elements are omitted for clarity and conciseness.

Of course, the embodiments of the invention can be implemented in a variety of architectural platforms, operating and server systems, devices, systems, or applications. Any particular architectural layout or implementation presented herein is provided for purposes of illustration and comprehension only and is not intended to limit aspects of the invention.

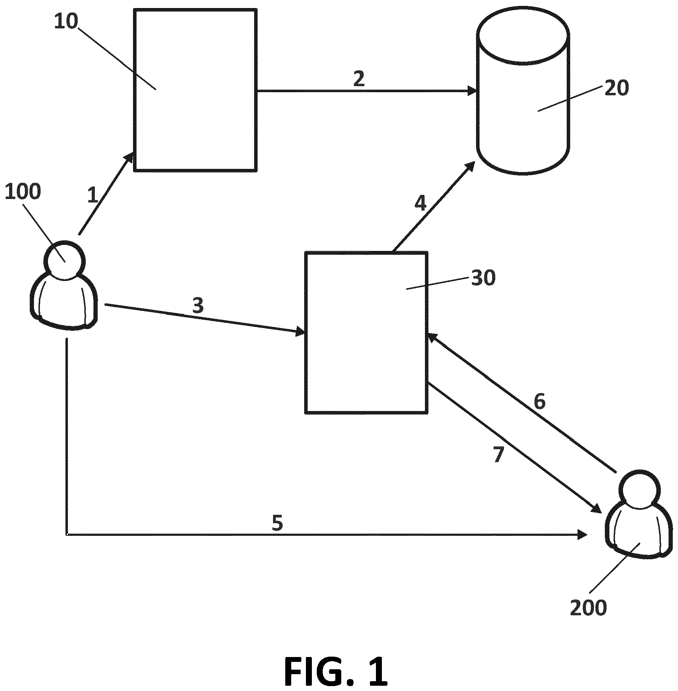

Figure 1 shows an overview of a system for designing and developing treatment plans according to a possible embodiment of the invention, wherein the following steps are performed in order to move the power of program creation from the engineering organisation to the treating organization and separate their concerns:
Step **1**: A treatment plan for a specific mental disorder is developed by a therapist **100** using a Content Editor **10.**
Step **2:** The edited plan ready to be used is stored in a Content Storage **20.**
Step **3**: An account for the patient **200** is configured by the therapist **100** using an Administrator panel **30.**
Step **4:** The Administrator panel **30** fetches from the Content Storage **20** a specific plan assigned by the therapist **100** to the new patient **200.**
Step **5:** The patient **200** is informed about the availability of the plan from his/her therapist **100,** providing for instance an activation code or a password if the plan is restricted for public access.
Step **6:** The App or computer program running in a patient's equipment, e.g., smartphone or personal computer, contacts the Administrator panel **30** by providing the activation code/ password that identifies the patient **200.**
Step **7:** The Administrator panel **30** responds to the request received from the App/computer program, for example after validating the activation code/ password received from the App/computer program within said request, and the Administrator panel **30** activates or download the correct plan for the patient **200.**

The proposed system comprises four layers:
i) The static layer, which is a file with data content defined by a specialist, the therapist **100,** using a predefined machine readable format (e.g., the JavaScript Object Notation, JSON format; Extensible Markup Language, XML; or custom formatting) to create and edit the treatment plan description.
ii) The data modelling layer, which reads the static file containing the treatment plan and transforms it into a logical model which a computer program can work with.
iii) The application logic layer, which works with the generic logical models created by the data modelling layer and ensures that the patient actions triggered for the content defined at the static layer are translated into events. This application logic layer either alters the treatment plan dynamically based on the generated events or registers these events to be interpreted by a practitioner or a machine.
iv) The visualisation layer presents the models created at the data modelling layer (and potentially modified at the application logic layer) to the patient **200** in a visual way and offers ways for the patient **200** to interact with the model, so that actions can be detected and acted upon as described in the application logic layer.

One of the greatest benefits comes from allowing the same visual design across various apps. Colors and images can naturally change but the overall features and navigation of the system stays the same. In this scenario, only the program file at the static layer i) is replaced, but the rest of the layers ii)-iv) stays completely unchanged. This allows the therapist **100** to design a program for an unsupported condition without requiring any engineering efforts.

In a preferred embodiment, the visualisation layer can be built using visual components, which are isolated software blocks, each focused on representing one part of the user experience (e.g., anything from a screen with some text to an interactive exercise). A user facing feature is built from layers of visual components in layer iv). In an example, a visual component can be a self contained feature. In another example, a visual component "component-c" can be built on another visual component "component-b" that is, in turn, built on a visual component "component-a".

For some treatment plans, a more specialized user experience might be required, especially on a higher level and a complete reuse of the visual layer might not be feasible. In this scenario, also partial component reuse is an option. While the main application flow and experience might change, the majority of the underlying generic components may still be reused. The layered visual architecture allows for a great level of flexibility at the lowest cost possible. Thanks to the complete separation of the user facing feature from the rest of the architecture, it is also possible to build a completely different App replacing all of the visual components in Layer iv) and replacing it with something completely new. The good thing is that while the user facing feature might need a complete rework and lots of attention, the other layers ii)-iii) can stay unchanged.

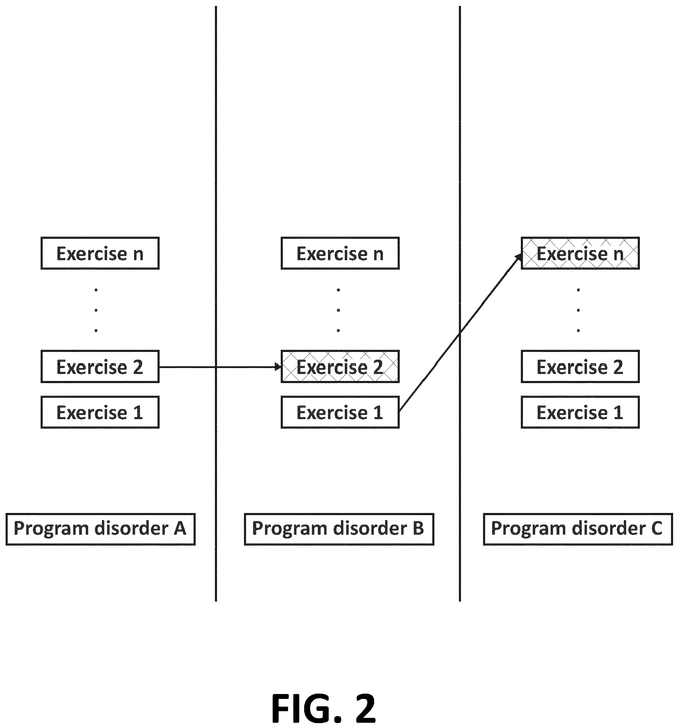

Furthermore, since the data modelling layer ii) sets the rules for how the program can be declared, reusing static content between programs in layer i) is a completely viable option. This means that exercises created for a treatment plan or disorder **program A** can be cross referenced from treatment plans or programs for other disorders. Figure 2 exemplifies how **Exercise 2** is referenced from **program B** and how Exercise 1 in **program B** is referenced as **Exercise n** in **program C,** having **programs A, B and C** defined for different mental disorders.

The technical structure of the proposed systen mimics a more traditional treatment plan as offered by the therapist **100** and starts off with an encapsulating container called disorder program. The disorder program is what encapsulates everything that the therapist **100** would offer the patient **200** during months of sessions. A disorder program is focused on talking the patient **200** through a structured and ordered plan for treating a mental condition such as depression. The outline of a disorder program description could look like (but is not restricted to) the computer program implemented description below, e.g., in JSON format (in this example, the disorder program only contains a single Block and a single Exercise):

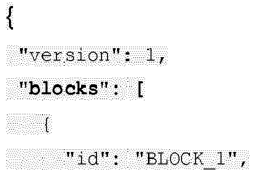

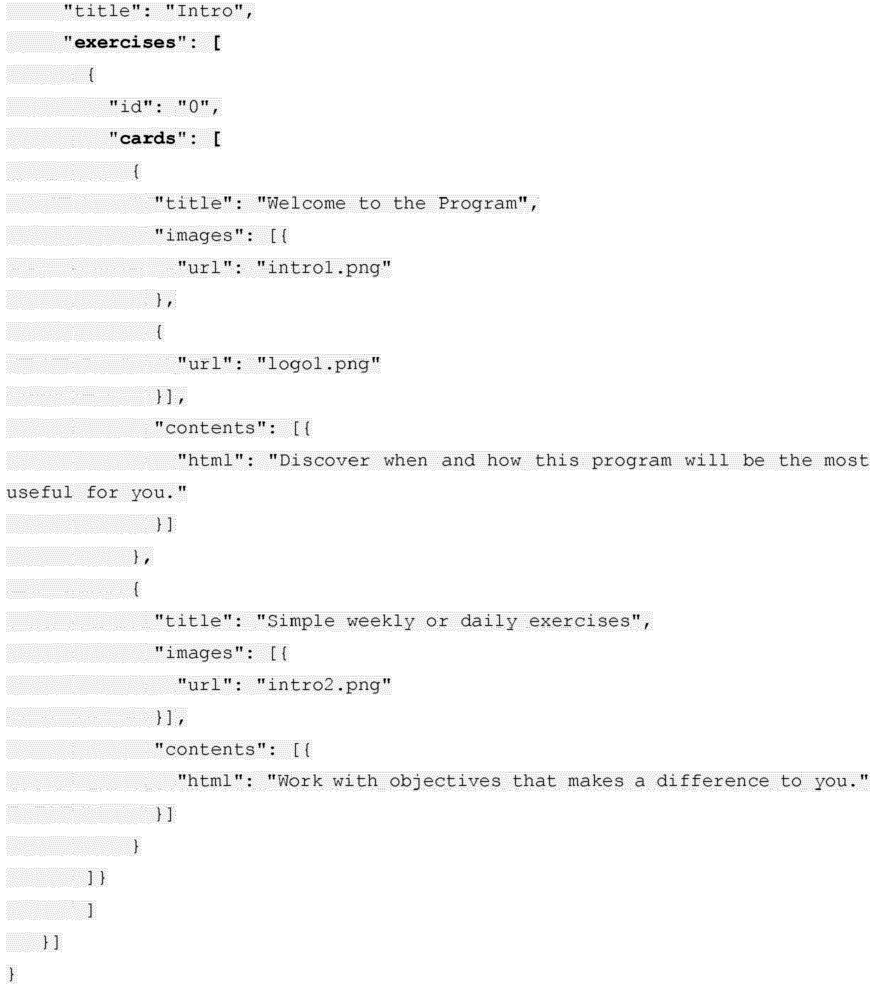

This tree structure of the disorder program that is grouped into Block and then Exercises and Cards provides the therapist **100** with a well defined way to create a linear or even nonlinear treatment plan build into different steps. The details and exact parameters for each building block of the disorder program can naturally vary over time and with the complexity of the disorder program. A possible embodiment of the invention proposes the elements listed in Table 1 below as a good starting point:

**Table 1**

| | |
|---|---|
| Version | Version of the program to handle changes and updates |
| Blocks | An array or list of components steps grouping the treatment plan into logical groups. |

A disorder program contains one or more Blocks. A Block is a container for treatment segments that creates a logical group of exercises offered to the client as part of the treatment. A Block has at least the following characteristics listed in Table 2 below:

**Table 2**

| | |
|---|---|
| Optional | Some blocks are required for the patient to complete while others might not apply to the specific circumstances. The therapist or the patient himself is able to exclude non-mandatory blocks from the Program by configuration or by interactive selection for instance when the application is started the first time. |
| Type | The type of block, making it possible for instance to only show certain blocks once, until they have been completed and then they are not visible anymore or any other kind of special handling needed by the App that visualizes the content to the patient. |
| Title | Block title |
| Description | Block description |
| Exercises | An array or a list of exercises encapsulated by this block. |

A block typically encapsulates a series of Exercises logically grouped together. For a condition that involves asocial behaviour, a typical block could contain exercises gradually encouraging the patient to leave the home. An Exercise has at least the following characteristics listed in Table 3 below:

**Table3**

| | |
|---|---|
| Title | Exercise title |
| Description | Short description about the purpose of the exercise |
| images | Images related to the introduction of the exercise. Covers or similar. |
| is Repeatable | Can the patient repeat the exercise |
| isPostponable | Can the patient postpone doing the exercise |
| cards | Array or list of cards, each card describing a step in the exercise. |

A Card is a generic concept that describes a part of an exercise. An exercise is build from one or more cards, each fulfilling a specific purpose. The high level attributes of a card are similar to the ones in Exercise or Block. Some examples of Card content are the ones listed in Table 4 below:

**Table 4**

| | |
|---|---|
| Title | Card title |
| Subtitle | Subtitle |
| Images | Images(s) |
| Content | Content for instance in html format. |
| Checklists | Questionnaires with single/ multi option replies from predefined sets |
| Textareas | Questionnaires with free text input |
| Quiz | Interactive questionnaires with immediate feedback |
| Media | Interactive media such as video or audio exercises. |

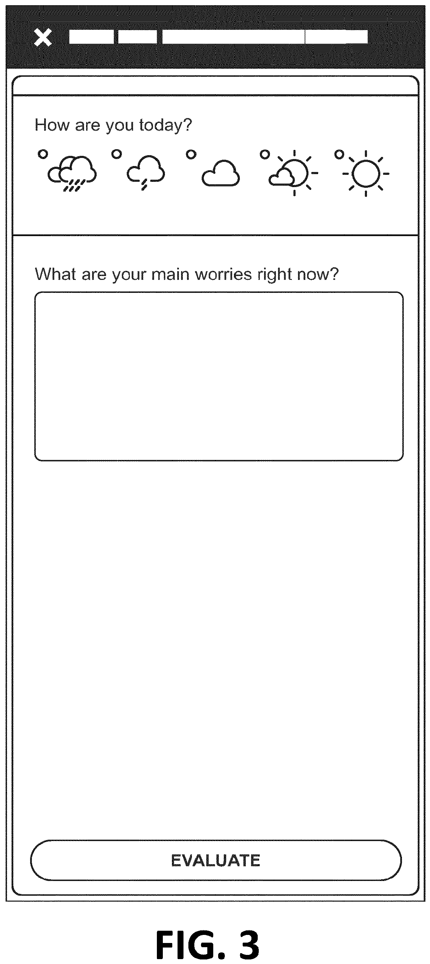

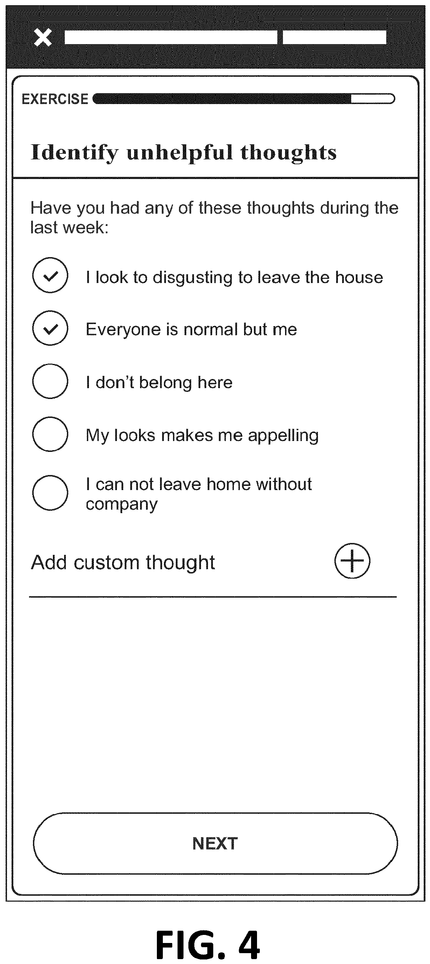

The characteristics listed in Table 4 above are enough to display static information such as an explanation or when giving instructions to the patient **200** but in the end it is little more than a vaguely interactive book. The main advantages of using the dynamic approach is that an app can give different ways of information presentation if more advanced cards can be defined in the program file. Some examples are:
- Audio cards: By declaring a link to an audio file in step **1,** the therapist **100** defining the program content will be able to present the patient **200** with an audio exercise. Steps **2** to **4** in the applications are already prepared to model, interpret and visualize the audio card so all what the therapist has to do when defining the exercise and its corresponding cards is to declare on card which audio properties will automatically render to the user.
- Video cards: Video cards are similar to audio cards. The therapist **100** only has to define one or multiple cards in an exercise as video cards and the end result for the patient **200** is that a therapeutically video is presented when that step of the Exercise is reached.
- Content cards: Content cards could be seen as a standard web page containing explanatory texts, images and other static content.
- Advanced Cards: The types of cards given as examples above (audio, video, content) solve specific and static use-cases. This goes a long way towards providing the patient with one-way information based on the content in the static layer, but will not provide the interactive and dynamic parts that are needed in order to take the treatment to the next level. In order to do that, the concepts of textareas and checklists in cards are introduced here in order to collect user input. These components can then be arranged in the desired way in an advanced card.
   ∘ Textareas: In some exercises the patient **200** needs to input provide free-text input to the exercise. This is done using a textarea component at Layer i), as shown in Figure 3, and the results are stored with a unique identifier so that the answer can be retrieved later on. The answers can then be used in other exercises, for self-reflection or for analysis by the therapist. An example of a declaration of an array of textareas for user input, in JSON format, is shown below: 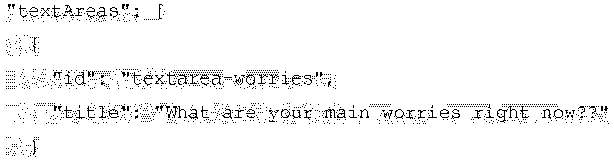 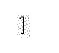
   ∘ Checklists: During certain phases or checkpoints in the Program, it is important to collect feedback from the patient **200** or use the patients' thoughts or reflections as input to future exercises. A straightforward way to collect patient's input is to display predefined checklists from where the patient **200** can select the best fitting options.
      In the simplest scenario the program file at layer i) declares a static list of options that should be presented to the patient **200** on a given card. The example card simply has a title and a declaration for the text for the button leading to the next page (nextButtonText) and inside it contains a single checklist asking for user input. An example of a simple static checklist asking the patient **200** about their negative thoughts during the last couple of days is shown below: 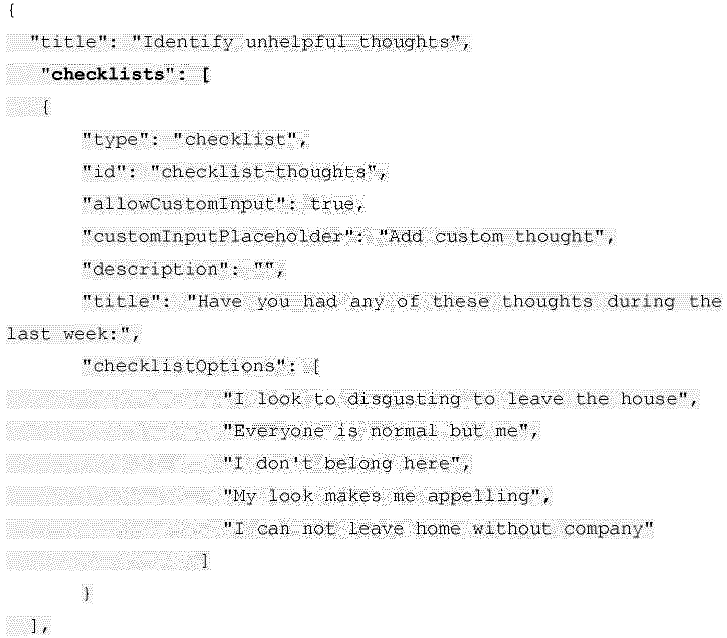 The declaration above will render into the image visualized by layer iv) as shown in the example of Figure 4. A checklist where the patient **200** can choose from the various options statically defined.
      There are several ways to connect user input across exercises at any point in time during the patient journey through the disorder program. In order to accomplish this, an option of implementation is to uniquely identify previous answers by assigning a unique identifier to the individual checklist, for example: 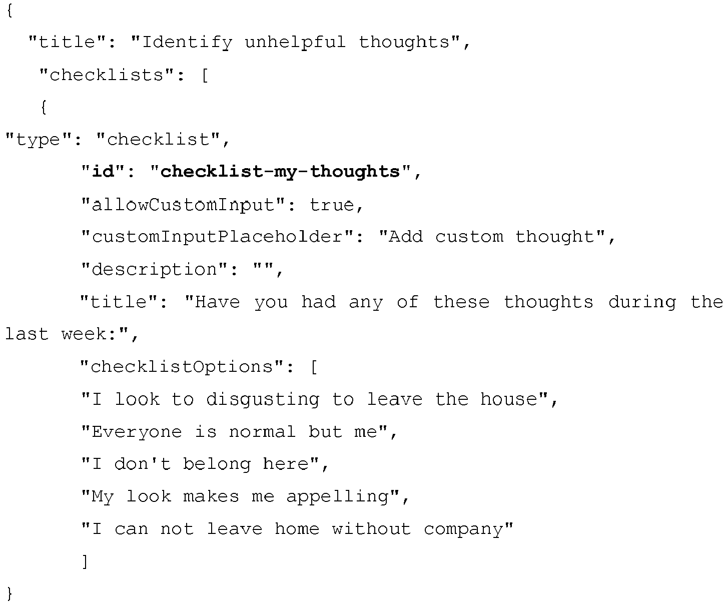 Thus, when the patient **200** selects from the available options of the checklist shown in Figure 4, the responses are stored based on the checklist identifier and the responses can be retrieved and used in future exercises.

With the possibility to retrieve previous input from the patient **200,** new additional cards can be generated referring back to the replies in a previous exercise. Dynamically generated cards could be declared based on previous patient input as the checklist declared below:

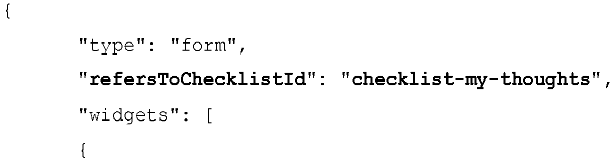

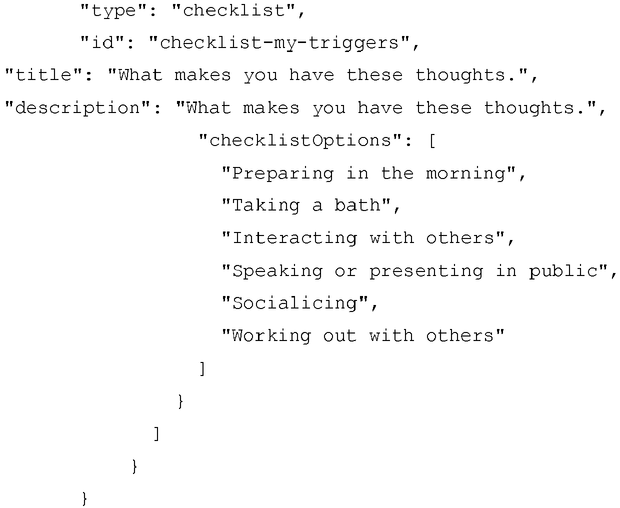

This card introduces the "referesToChecklistld" parameter and the effect is that while declared in the static layer i), this layer triggers the application logic layer iii) to generate new cards dynamically based on the patients input on the first card. Each answer from the "checklist-thoughts" card will generate one dynamic card asking for the triggers of the initial thoughts in this example.

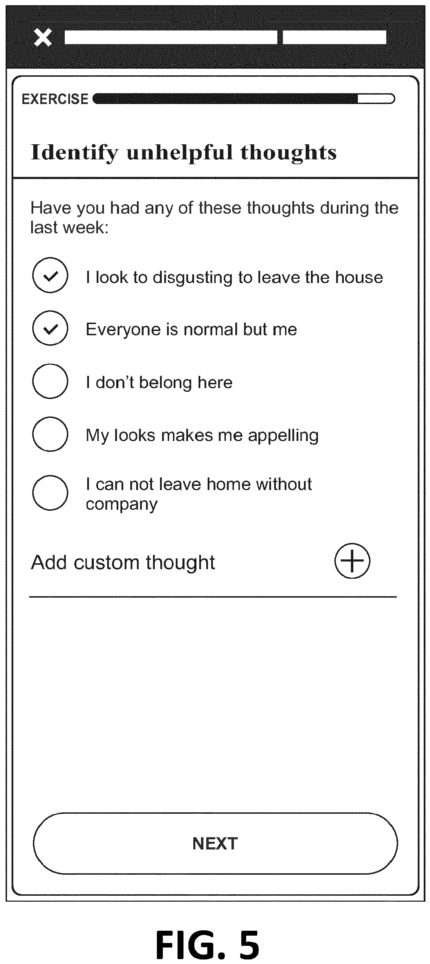

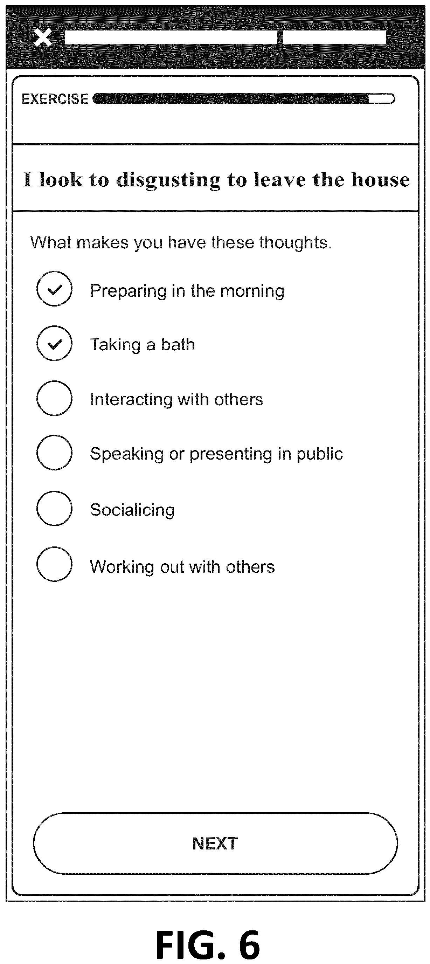

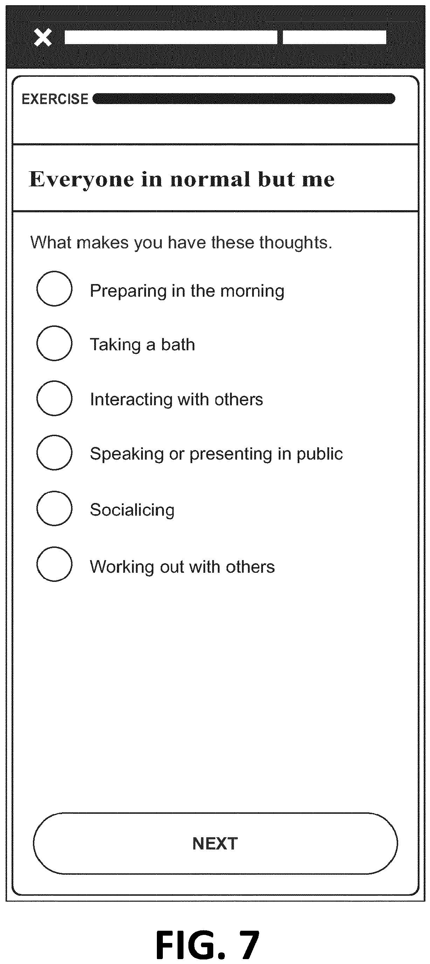

Figure 5 shows a scenario where two options for the card generator are selected. When processing the following card the application logic layer iii) detects that the card declares the "refersToChecklistld" parameter referencing the generator cards "checklist-thoughts". This triggers the automatic generation and injection of two new cards (one for each option previously selected) into the deck of cards in the current exercise:
- Rendering in the visual layer iv) of the first dynamically generated card from the "I look disgusting..." option, shown in Figure 6.
- Rendering in the visual layer iv) of the second dynamically generated card from the "Everyone's staring" option, shown in Figure 7.

Here it is important to notice that only the first "template" for the cards to be dynamically generated is declared in the static layer i). If multiple answers are provided by the user in the initial questionnaire (checklist-thoughts), then multiple dynamic cards are generated automatically. In this case each dynamic card has a referring title which is the option previously selected and then a static list of new options that are used to further drill into the exercise and understand the patient. Also note that the card that declares this behaviour in the static layer i), also gives an identifier to the new checklist used on the dynamic cards ("checklist-triggers") preserving also these answers for future usage allowing the therapist **100** to dig deeper and deeper by progressively asking only the relevant questions based on previous patient input.

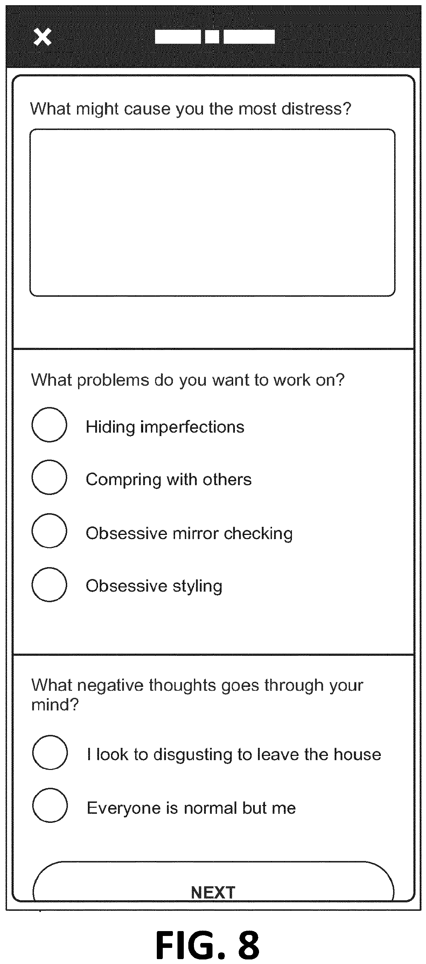

Another important scenario for the treatment plan (disorder program) to be efficient is to refer back to previous patient input at certain checkpoints in the program by dynamically generating follow up options. This scenario is different from the previous one since here the previous answers are provided as a dynamic checklist in order to reflect back, instead of generating a new card based on static options. In order to accomplish this new behaviour, the following actions need to be taken:
- Card that generates checklist options from previous answers at the static layer i)
   The static layer i) introduces a new parameter called for instance "getOptionsFromChecklistld". In the example below a Card inside an Exercise declares an array of Checklists (only the checklist array is shown for clarity). The first checklist contains a list of static options for the patient **200** to select from. The second checklist however, instead of specifying its own options, declares the "getOptionsFromChecklistld" that indicates that the options for this list needs to be retrieved from previous replies and injected into this card. 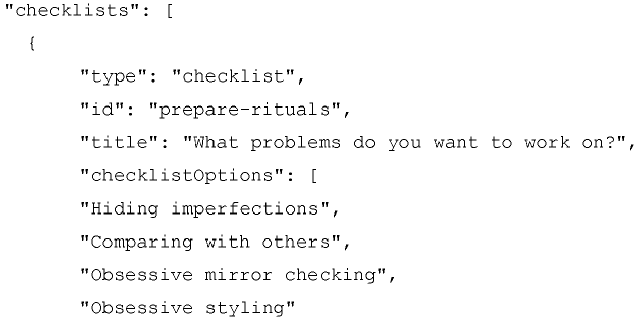 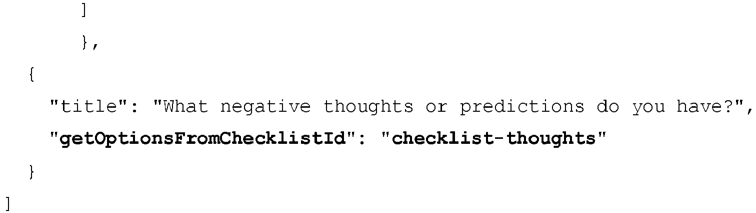
- Injecting options in existing Card at the application logic layer iii)
The logic layer iii) dynamically creates and injects checklist option into the questionnaires on the declared Card in order to ask the patient **200** relevant questions following up on previous selections. Figure 8 shows how the "getOptionsFromChecklistld" has been used by the logic layer iii) to automatically inject checklist options into a Card. First of all both checklists from the static layer i) are visualized as lists of selectable options. The first one contains nine static options from the first list declared above while the second list contains the two options previously provided by the patient **200** in an earlier exercise (and stored in checklist-thoughts).

This approach allows the therapist **100** to refer back to previous input from the patient **200** and create follow up questions at different point of the treatment and in different contexts, something that is not possible to accomplish using a static treatment plan. This way the therapist **100** can form instance see how the patients' replies to the same questions evolve over time, without requiring any new development from the engineering team. Any question can be referred back to at any time.

In some scenarios cards with advanced or extensive content needs to be declared in advanced in the static layer i) but then hidden based on patient **200** input in previous exercises. This is due to that some content might not apply in a specific exercise depending on what the patient **200** has responded earlier. This scenario is resolved by introducing a filter parameter in the static layer i) which allows the person that creates the program to introduce dynamic filtering of the content without the need of any custom development.

In the example below three filtering parameters are introduced for the card with id="describe-defaults". The "filterByChecklistld" refers back to a checklist answered by the patient **200** in a previous exercise. If the "filter" phrase is in the set of answers provided by the patient **200,** this card will be shown in order to further work with this issue. If the patient **200,** in this case, does not identify himself as defective, the exercise will not focus on further evaluating that belief. Finally this card uses the "filterType" parameter with the value "aggregated". This means that all the patients' answers for this exercise over time will be used by the filter and not only the latest replies. This allows the program designer to take into consideration that a patient **200** has repeated the exercise leading up to this one several times and potentially providing different answers along the progress of the treatment. Aggregating the answers allows for evaluation of all the beliefs compared to just a subset such as the last ones if needed. Below, there is an example of a card declaration in the static layer i) that allows filtering out cards based on previous patient input:

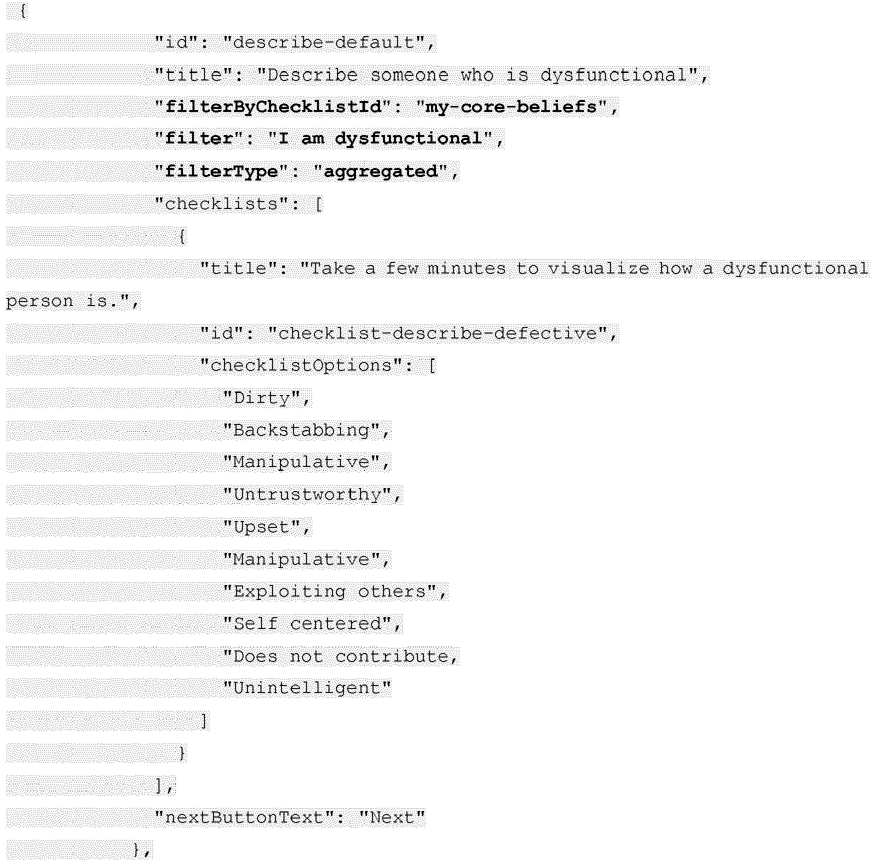

### Dynamic content injection

Even though many cards will provide static content to educate the patient 200 about the condition, the content is perceived as much more personalized if replies from the patient 200 are seamlessly injected into the educational cards. This is accomplished using another tag called "generateFromChecklistld" that dynamically creates a visual representation of the replies in a previous exercise and injects it into the current card. Below, there is an example of the declaration of a card that generates dynamic lists at the static layer i) from patients answers in previous exercises:

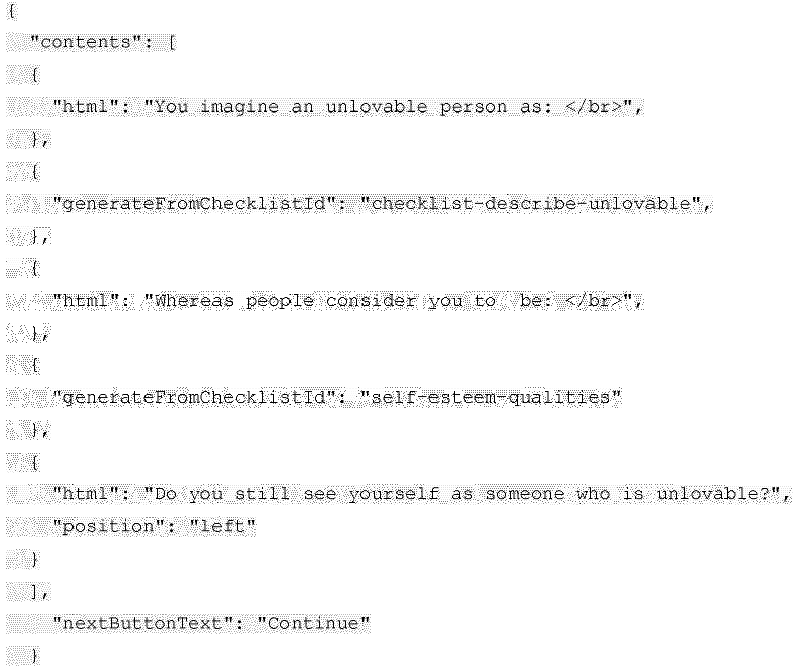

Some parts of the content will have to change depending on the patient **200** and for instance the hospital. One example is the therapist **100** managing the treatment. In order to give the program a personal and credible feeling, a patient **200** must be assigned a therapist **100.** This can be a person that has previously treated the patient or someone only available remotely. Either way, the patient **200** needs to be presented with some personal information about the therapist **100** such as a photo and a bio. This information cannot be part of the actual treatment program file since not all clients will use the same therapist **100** and the same program can be reused in different hospitals. So in order to achieve this personalization, the therapist's info (and other dynamic content) is injectable during runtime by the logic layer iii). In order to be able to inject generic dynamic content, modifications are needed on both the static layer i) and the application logic layer iii).

First of all the invention introduces the concept of a unique tag with a dynamic content identifier. In this example, three angle brackets are used and inside the name of the unique identifier is declared. In the example below, the unique identifier is called coach-bio and it is marked up using [[[]]] brackets, but naturally other systems might choose different tags. An example of an exercise statically declaring a single card at layer i) with a single generic content element injection is as follows:

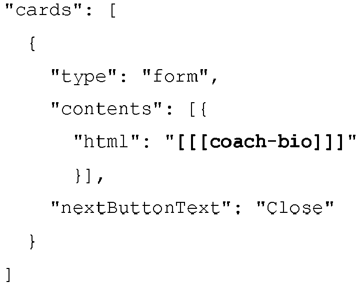

In order to be able to read and inject dynamic content the logical layer iii) introduces a dynamic content storage. This is a generic read/write repo that has an application program intermace (API) similar to the one below: setDynamicContent(dynamicContentld: string, content: string) {} getDynamicContent(dynamicContentld): string {}

This allows any part of the application to register dynamic content, for instance in html format, to the dynamic content service. The when setting up the program or when preparing the next card the logic layer needs to scan through all content elements to see if there is any content that needs to be dynamically injected. If so, the data in the html tag above will be replaced by the content retrieved from the getDynamicContent method passing the unique identifier found in the static program file.

The [[[coach-bio]]] tag declared in the static layer i) can be transformed into a visual representation of how the therapist's bio is displayed to the patient at visualization layer iv). The actual html data shown is read from another source which is not related to the program file. This approach allows different data sources to be dynamically combined with the treatment plan without interfering with its content. The therapist **100** is responsible of and can focus on the program creation. The patient **200** knows that a therapist will be assigned to him/her and simply declares in the program file that this info should be shown on a certain page and does not have to worry about the details of how that works, neither will the therapist **100** have to request additional development to be done. This method can naturally be applied to any kind of content that is provided as part of the treatment bundle to the user and then injected into the application.

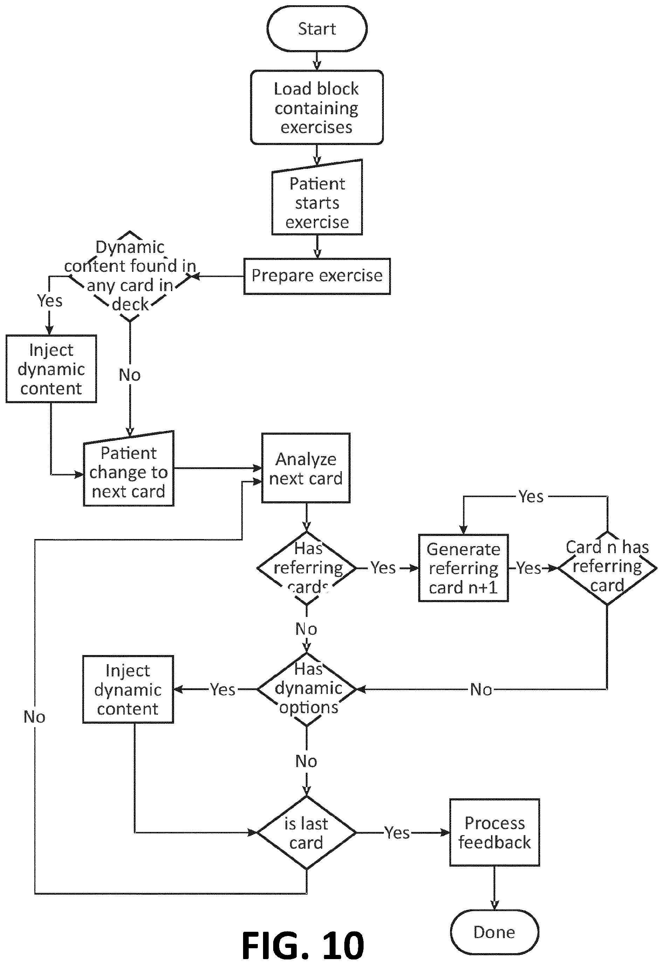

Figure 10 illustrates a very high level flow, focused primarily on how the dynamic content is managed, of an exercise being loaded and worked on by the patient **200.** The block containing the exercises for a specific treatment plan assigned by the therapist **100** to the patient **200** is loaded in his/her user terminal (e.g. mobile terminal or personal computer) so that the patient **200** can start one or more of the exercises. In order to prepare an exercise, the patient **200** finds a deck of generated cards and check whether dynamic content can be found in any card of the deck. If so, the dynamic content is injected into a next generated card; otherwise, the patient **200** is prompted to change from the current card to a next generated one. The generated next card is analyzed to check whether the card is referring to other cards and if so, all the referred cards are generated for the exercise. Then, the generated next card is analyzed to check whether the card has dynamic options and if so, the dynamic options are injected into the card. The process is repeated until the patient **200** gets the last card of the deck and finally a feedback based on the patient's answers is generated.

Note that in this text, the term "comprises" and its derivations (such as "comprising", etc.) should not be understood in an excluding sense, that is, these terms should not be interpreted as excluding the possibility that what is described and defined may include further elements, steps, etc.

## Claims

1. A computer-implemented method for generating treatment plans of psychological illness and disorders, **characterized by** comprising:
- generating, at a static layer, a file containing data static content which declares a treatment plan using a machine readable format, the treatment plan defined by a therapist (100) for a patient (200),
- reading the static content of the file and creating, at a data modelling layer, based on the static content, a logical model with which a computer program works;
- generating, at an application logic layer, events associated with inputs from the patient (200) which are triggered for the static content defined at the static layer, the events being used to create dynamic content which is either stored or used to modify dynamically the logical model created at the data modelling layer;
- presenting the logical model, at a visualisation layer, to the patient (200) and receiving the inputs from the patient (200).

2. The method according to claim 1, wherein generating the file at the static layer comprises declaring at least a block, using the machine readable format, the block being a container encapsulating a series of exercises, logically grouped as a part of the treatment plan and defined by the therapist (100), to trigger the inputs from the patient (200).

3. The method according to claim 2, wherein generating the file at the static layer further comprises declaring at least a first card, using the machine readable format, the first card having the static content containing a part of one exercise of the series of exercises to trigger inputs from the patient (200) and the first card having at least an attribute used by the visualisation layer to present the logical model.

4. The method according to claim 3, wherein the attribute of the first card is a link to an audio file.

5. The method according to claim 3, wherein the attribute of the first card is a link to a video file.

6. The method according to claim 3, wherein the attribute of the first card is a link to a web page.

7. The method according to claim 3, wherein the attribute of the first card is a textarea or a checklist.

8. The method according to any of claims 3-7, wherein creating dynamic content comprises storing the received previous inputs from the patient (200) triggered by the first card and further comprising generating at least an additional card, declared at the static layer using the machine readable format and used at the application logic layer to modify the logical model, the additional card having the dynamic content created from the events associated with the stored previous inputs from the patient (200) and having at least an attribute used by the visualisation layer to present the modifed logical model, the attribute being selected from a link to an audio file, a link to a video file, a link to a web page, a textarea and a checklist.

9. The method according to any preceding claim, wherein the machine readable format is JavaScript Object Notation, JSON or Extensible Markup Language, XML.

10. A system for generating treatment plans of psychological illness and disorders **characterized by** comprising a processor implementing four layers, a visualisation layer built on an application logic layer which is built on an a data modelling layer, in turn, built on a static layer, and a content storage connected to the processor, the processor further comprising:
- a content editor (10) with means for generating, at the static layer, a file containing data static content which declares a treatment plan using a machine readable format, the treatment plan defined by a therapist (100) for a patient (200);
- means for reading the static content of the file and creating, at the data modelling layer, based on the static content, a logical model with which a computer program works;
- means for generating, at the application logic layer, events associated with inputs from the patient (200) which are triggered for the static content defined at the static layer, the events being used to create dynamic content which is either stored in the content storage (20) or used to modify dynamically the logical model created at the data modelling layer;
- a user interface for presenting the logical model, at the visualisation layer, to the patient (200) and receiving the inputs from the patient (200).

11. The system according to claim 10, wherein the content editor (10) uses either JavaScript Object Notation, JSON, or Extensible Markup Language, XML.

12. The system according to any of claims 10-11, wherein the content editor (10) generates the file at the static layer by declaring at least a block, using the machine readable format, the block being a container encapsulating a series of exercises, logically grouped as a part of the treatment plan and defined by the therapist (100), to trigger the inputs from the patient (200).

13. The system according to claim 12, wherein the content editor (10) generates the file at the static layer by further declaring at least a first card, using the machine readable format, the first card having the static content containing a part of one exercise of the series of exercises to trigger inputs from the patient (200) and the first card having at least an attribute used by the visualisation layer to present the logical model.

14. The system according to claim 13, wherein the user interface comprises an audio player to play an audio file linked to the attribute of the first card.

15. The system according to claim 13, wherein the user interface comprises a video player to play a video file linked to the attribute of the first card.

16. The system according to claim 13, wherein the user interface comprises a multimedia player to play multimedia content of a web page linked to the attribute of the first card.

17. The system according to claim 13, wherein the user interface comprises a screen to display a textarea or a checklist defined by the attribute of the first card.

18. The system according to any of claims 10-17, wherein the processor is further configured to create dynamic content from the events associated with the previous inputs from the patient (200) triggered by the first card and stored in the content storage (20) and further comprising generating at least an additional card, declared at the static layer using the machine readable format and used at the application logic layer to modify the logical model, the additional card having the dynamic content and having at least an attribute used by the visualisation layer to present the modifed logical model, the attribute being selected from a link to an audio file, a link to a video file, a link to a web page, a textarea and a checklist.
